# EUROPEAN PATENT APPLICATION

(11) **EP 4 767 969 A1**
(43) Date of publication of application: **01.07.2026**
(21) Application number: 25221005.9
(22) Date of filing: 05.12.2025
(51) Int. Cl.: A61B 18/14

(54) **PLANAR CATHETER WITH SENSING LOOPS AND ELECTRODES ON THE SAME FLEXIBLE CIRCUIT**

(30) Priority: 31.12.2024 US 202419007222
(71) Applicant: Biosense Webster (Israel) Ltd., 2066717 Yokneam (IL)
(72) Inventor: EBRAHIMI, Babak, Irvine, 92618 (US); VAN NIEKERK, Pieter Emmelius, Irvine, 92618 (US); ABBAS, Mohammad, Irvine, 92618 (US); RODRIGUEZ SOTO, Juan, Irvine, 92618 (US)
(74) Representative: Carpmaels & Ransford LLP

(57) **Abstract**

The disclosed technology includes a flexible circuit for an end effector of a medical probe. The flexible circuit includes a flexible substrate, electrodes, and a first sensing loop. The flexible substrate defines tines extending along a longitudinal axis. The electrodes are disposed on each of the tines. The first sensing loop is disposed on the flexible substrate. The first sensing loop forms one or more coils that generate a current that is indicative of a position of the one or more coils when the one or more coils is subjected to a magnetic field. The first sensing loop includes two segments disposed on respective tines extending generally parallel to the longitudinal axis and converging towards each other on a tine disposed on the longitudinal axis.

## Description

### FIELD

The present technology relates generally to medical devices, and in particular medical probes with electrodes, and further relates to, but not exclusively, medical probes suitable for use to map and/or ablate tissue.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Moreover, electrical traces and other components associated therewith can be prone to breakage and/or delamination when in use.

### SUMMARY

There is provided, in accordance with the disclosed technology, a flexible circuit for an end effector of a medical probe. The flexible circuit comprises: a flexible substrate defining a plurality of tines extending along a longitudinal axis; a plurality of electrodes disposed on each of the plurality of tines; and a first sensing loop disposed on the flexible substrate. The first sensing loop forms one or more coils configured to generate a current that is indicative of a position of the one or more coils when the one or more coils is subjected to a magnetic field. The first sensing loop comprises two segments disposed on respective tines, of the plurality of tines, extending generally parallel to the longitudinal axis and converging towards each other on a tine, of the plurality of tines, disposed on the longitudinal axis.

The is further provided, in accordance with the disclosed technology, a flexible circuit for an end effector of a medical probe. The flexible circuit comprises: a flexible substrate defining a plurality of tines extending along a longitudinal axis; a plurality of electrodes disposed on each of the plurality of tines; and first and second sensing loops disposed on the flexible substrate. Each of the first and second sensing loop form one or more coils configured to generate a current that is indicative of a position of the one or more coils when the one or more coils is subjected to a magnetic field. The first sensing loop comprises two segments disposed on respective tines, of the plurality of tines, extending along the longitudinal axis and converging towards each other on a tine, of the plurality of tines, offset from the longitudinal axis. The second sensing loop comprises two segments disposed on respective tines, of the plurality of tines, extending along the longitudinal axis and converging towards each other on another tine, of the plurality of tines, offset from the longitudinal axis.

There is further provided, in accordance with the disclosed technology, an end effector for a medical probe. The end effector comprises: an insulative material; a framework disposed in the insulative material, the framework being approximately planar along a longitudinal axis; and a first flexible circuit disposed in the insulative material such that the first flexible circuit is spaced apart from the framework along a vertical axis. The first flexible circuit comprises: a first flexible substrate defining a first plurality of tines extending along the longitudinal axis; a first plurality of electrodes disposed on each of the first plurality of tines; and first and second sensing loops disposed on the first flexible substrate. Each of the first and second sensing loop form one or more coils configured to generate a current that is indicative of a position of the one or more coils when the one or more coils is subjected to a magnetic field. The first sensing loop comprises two segments disposed on respective tines, of the first plurality of tines, extending along the longitudinal axis and converging towards each other on a tine, of the first plurality of tines, offset from the longitudinal axis. The second sensing loop comprises two segments disposed on respective tines, of the first plurality of tines, extending along the longitudinal axis and converging towards each other on another tine, of the first plurality of tines, offset from the longitudinal axis.

There is further provided, in accordance with the disclosed technology, a method comprising: forming a plurality of electrodes on a first surface of a plurality of tines of a flexible circuit, the plurality of tines extending generally parallel to a longitudinal axis; and forming a location sensing loop on the first surface. The location sensing loop forms one or more coils configured to generate a current that is indicative of a position of the one or more coils when the one or more coils is subjected to a magnetic field. The location sensing loop comprises two segments (i) running along respective tines, of the plurality of tines, and (ii) converging towards each other on a tine, of the plurality of tines, disposed on or offset from the longitudinal axis.

There is further provided, in accordance with the disclosed technology, an end effector for a medical probe. The end effector comprises: an insulative material; a framework disposed in the insulative material, the framework being approximately planar along a longitudinal axis; and a first flexible circuit disposed in or on the insulative material. The first flexible circuit comprises: a first flexible substrate defining a first plurality of tines extending along the longitudinal axis; a first plurality of electrodes disposed on each of the first plurality of tines; and first and second sensing loops disposed on the first flexible substrate. Each of the first and second sensing loop form one or more coils configured to generate a current that is indicative of a position of the one or more coils when the one or more coils is subjected to a magnetic field. The first sensing loop comprises two segments disposed on respective tines, of the first plurality of tines, extending along the longitudinal axis and converging towards each other on a tine, of the first plurality of tines, offset from the longitudinal axis. The second sensing loop comprises two segments disposed on respective tines, of the first plurality of tines, extending along the longitudinal axis and converging towards each other on another tine, of the first plurality of tines, offset from the longitudinal axis.

### BRIEF DESCRIPTION OF THE DRAWINGS

FIG. 1 is a schematic pictorial illustration of a medical system including a medical device that includes a medical probe with an end effector with electrodes, in accordance with the disclosed technology;
FIG. 2A is a schematic pictorial illustration showing an exploded perspective view of an exemplary end effector, in accordance with the disclosed technology;
FIG. 2B is a schematic pictorial illustration showing a cross-sectional view of the assembled end effector, taken along line L1 in FIG. 2A, in accordance with the disclosed technology;
FIG. 2C is a schematic pictorial illustration showing a similar cross-sectional view as that of FIG. 2B, with a modified configuration of the assembled end effector, in accordance with the disclosed technology;
FIG. 2D is a schematic pictorial illustration showing a similar cross-sectional view as that of FIG. 2B, with a modified configuration of the assembled end effector, in accordance with the disclosed technology;
FIG. 3A is a schematic pictorial illustration showing a plan view of a portion of a first flexible circuit of the end effector, in accordance with the disclosed technology;
FIG. 3B is a schematic pictorial illustration showing a cross-sectional view of a tine of the first flexible circuit, taken along line 3B-3B in FIG. 3A, in accordance with the disclosed technology;
FIG. 4 is a schematic pictorial illustration showing a plan view of the first flexible circuit, in accordance with the disclosed technology;
FIG. 5 is a schematic pictorial illustration showing a plan view of a second flexible circuit of the end effector, in accordance with the disclosed technology;
FIG. 6 is a schematic pictorial illustration showing a diagrammatic layout of where the detail views of FIG. 6A-6E are taken relative to;
FIG. 6A is a schematic pictorial illustration showing a detail view of a proximal portion of the first flexible circuit, in accordance with the disclosed technology;
FIG. 6B is a schematic pictorial illustration showing a detail view of an intermediate lateral portion of the first flexible circuit, in accordance with the disclosed technology;
FIG. 6C is a schematic pictorial illustration showing a detail view of a distal lateral portion of the first flexible circuit, in accordance with the disclosed technology;
FIG. 6D is a schematic pictorial illustration showing a detail view of a distal central portion of the first flexible circuit, in accordance with the disclosed technology;
FIG. 6E is a schematic pictorial illustration showing a detail view of a proximal central portion of the first flexible circuit, in accordance with the disclosed technology;
FIG. 7 is a schematic pictorial illustration showing a detail view of an alternative first flexible circuit, in accordance with the disclosed technology;
FIG. 8 is a schematic pictorial illustration showing a medical probe assembly, in accordance with the disclosed technology; and
FIG. 9 is a flow chart of a manufacturing method, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" or "generally" for any numerical values or ranges indicate a suitable dimensional tolerance that allows the part or collection of components to function for its intended purpose as described herein. More specifically, "about" or "approximately" or "generally" may refer to the range of values ±20% of the recited value, e.g. "about 90%" may refer to the range of values from 71% to 110%. For further example, "generally parallel" may refer to the range of values of parallel (i.e., 0 degree angle relative to one another) ±20 degrees. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

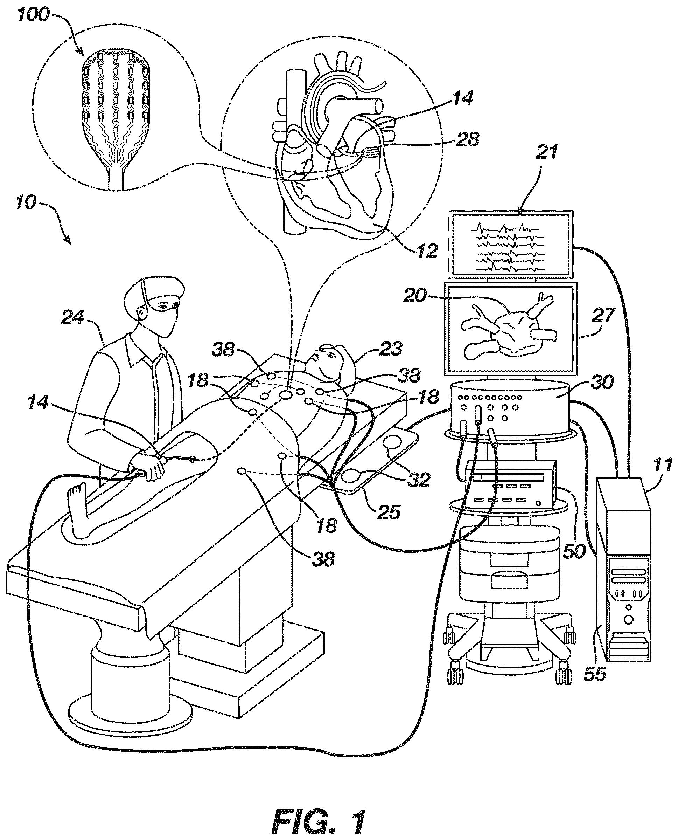

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter/medical probe 14 that is configured for sensing IEGM is illustrated herein. Physician 24 brings a catheter shaft with a distal tip 28 (*e.g.,* a multi-layered end effector 100) of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 102 optionally distributed over end distal tip 28 coupled to a catheter shaft and configured to sense the IEGM signals as described in more detail below. Catheter 14 may additionally include a position sensor embedded in or near end distal tip 28 for tracking position and orientation of distal tip 28. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 102. For impedance-based tracking, electrical current is directed toward electrodes 102 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 102 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 160A, 160B at an end effector of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

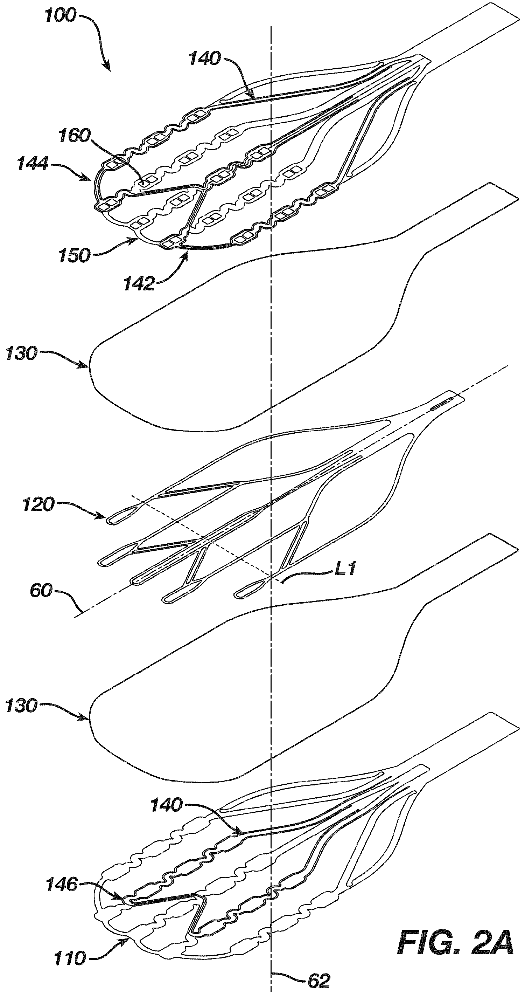

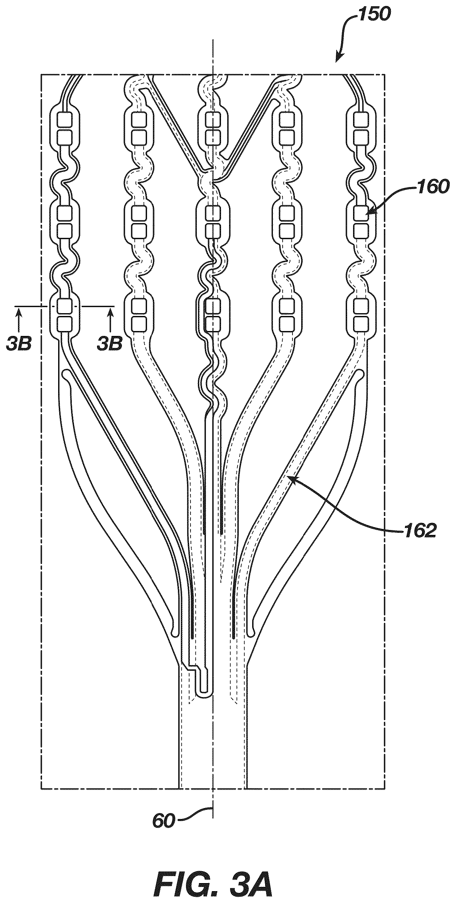

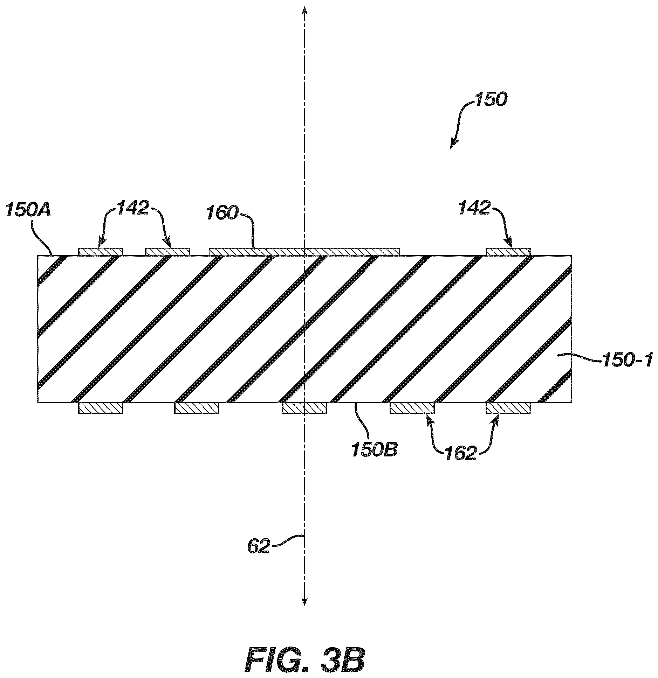

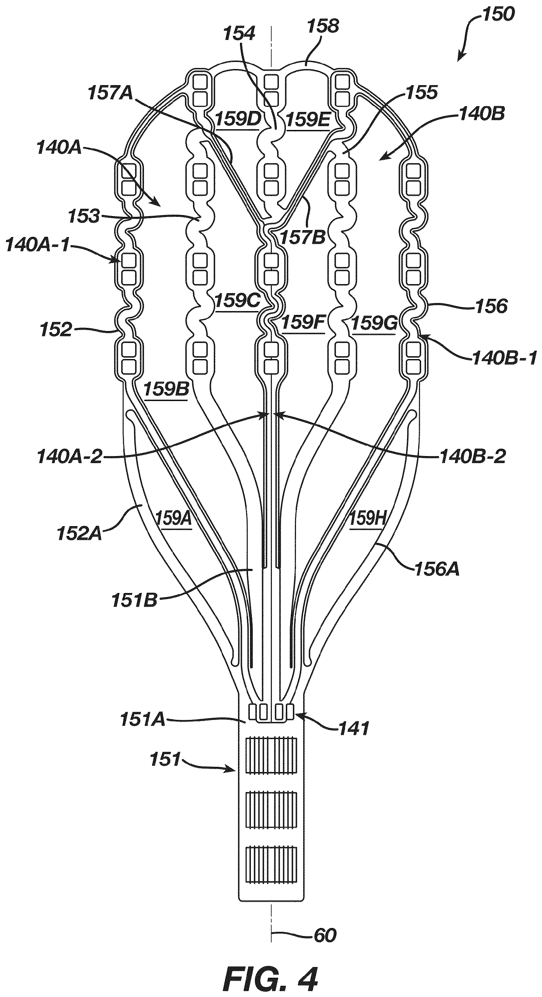

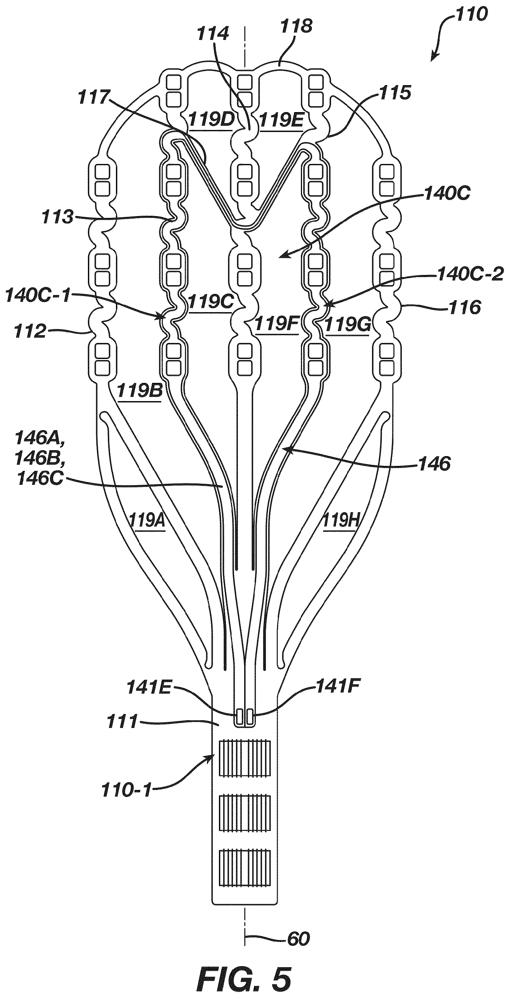

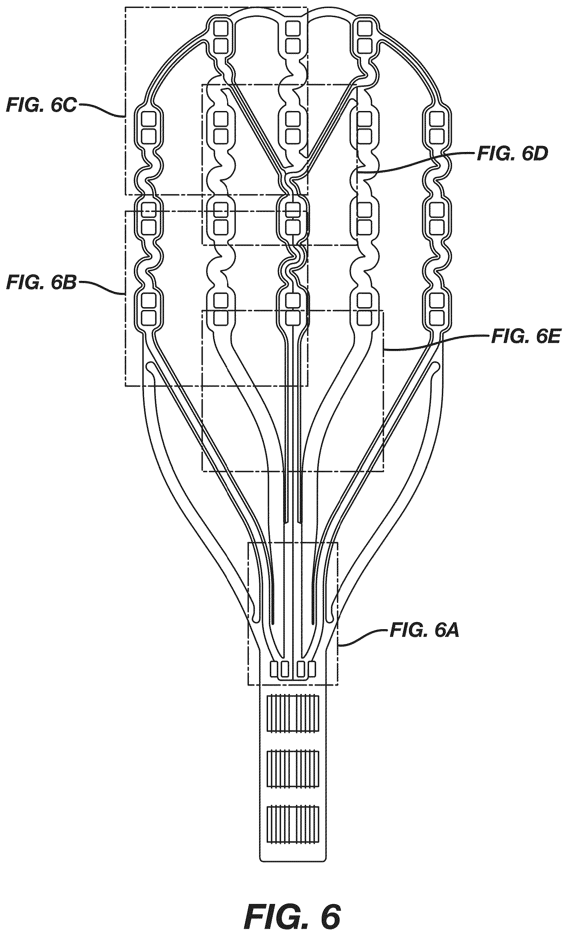

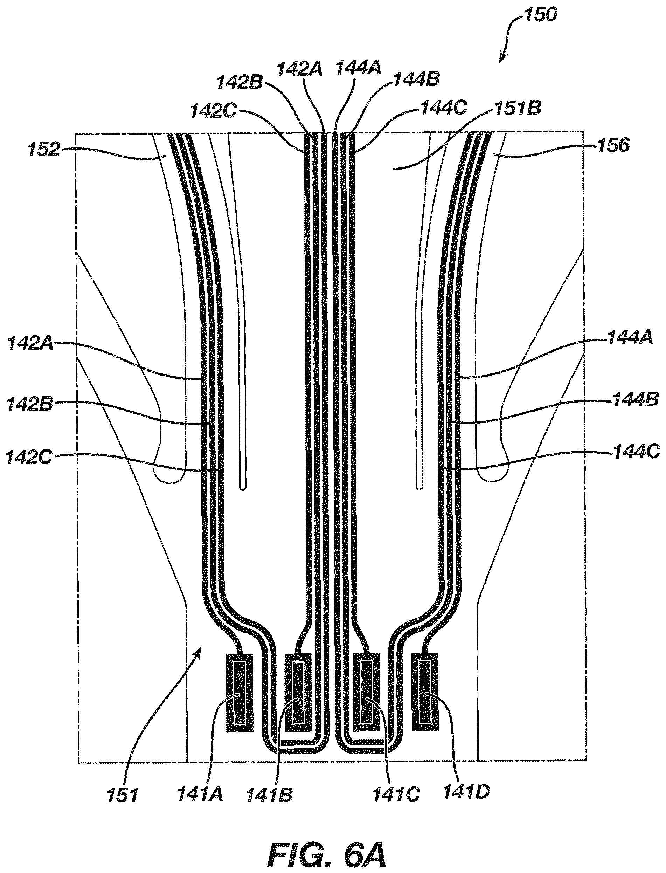

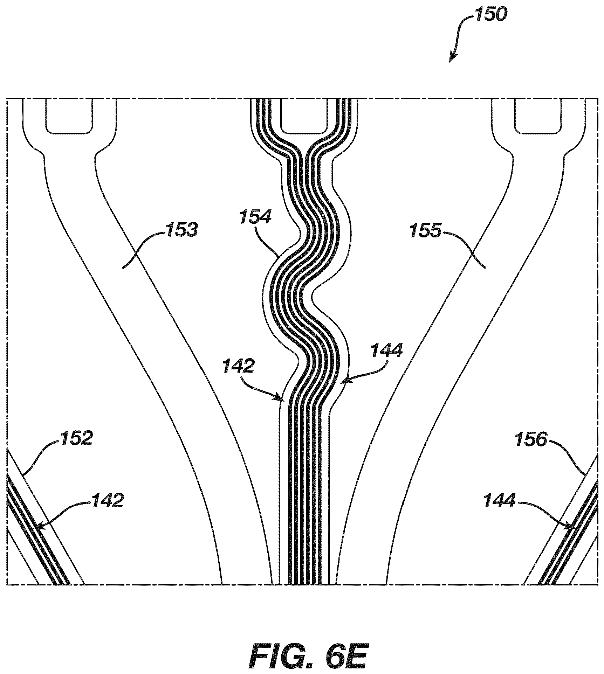

FIGs. 2A-6E provides various view of one or more portions of an end effector 100 (the term "end effector" is used synonymously with the term "distal tip" herein) that is configured for insertion into an internal body cavity of a patient. Specifically, FIG. 2A shows an exploded view of the first end effector 100, with the components thereof (it is noted that certain components, such as loop 146, are depicted for illustrative purposes, and are not necessarily disposed on the side/region depicted in FIG. 2A) extending along a longitudinal axis 60 and exploded vertically along vertical axis 62 (which is orthogonal to the longitudinal axis 60), FIG. 3A shows a portion of a flexible circuit 150 of the end effector 100, FIG. 3B shows cross-sectional view of the flexible circuit 150, cut along line 3B-3B in FIG. 3A, FIG. 4 shows the flexible circuit 150, the flexible circuit including one or more location sensing loops 140, FIG. 5 shows a view of another, opposing flexible circuit 110 of the end effector 100, and FIGs. 6A-6E are detail views of the flexible circuit 150. The opposing flexible circuit 110 is identically designed in this example, with the exception of the configuration of the location sensing loops 140, and, therefore, any description of the first flexible circuit 150 also accurately describes the configuration of the second flexible circuit 110, and vice versa, unless specifically noted to the contrary.

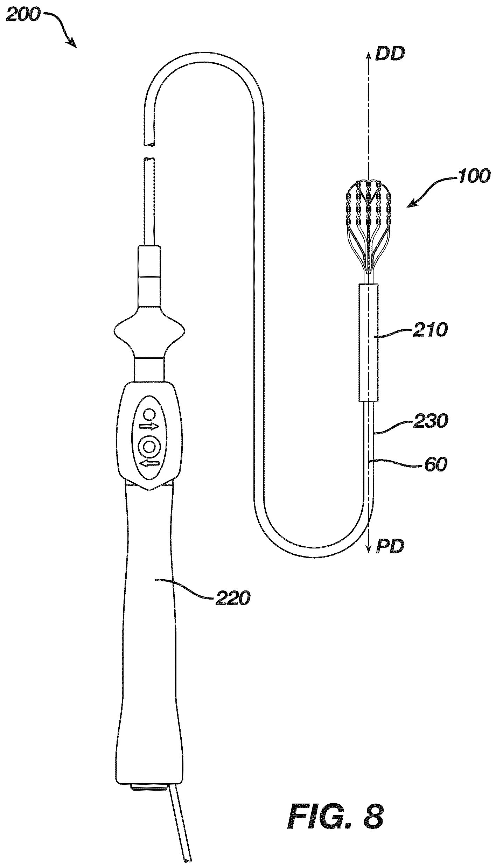

The end effector 100 extends from a proximal end (upper right-hand side of FIG. 2A), that connects to an elongated shaft 230 (FIG. 8 , also referred to as a tubular member), to a distal end (bottom left-hand side of FIG. 2A) along a longitudinal axis 60, and includes components contiguous with and/or disposed within an insulative material 130. For example, the end effector 100 can include a first flexible circuit 150 and a second flexible circuit 110. The first flexible circuit 150 (and the second flexible circuit 110) includes a plurality of electrodes 160. In some examples, the term "flexible circuit" includes thin-film circuit, flexible printed circuit board, thin film deposition via lithography and etching processes on substrates such as polyimide, copper, LCP, nitinol substrate, thermoplastic polyurethane (TPU), silicone, thermoset resin, or other polymeric substrates. In some examples, the flexible circuits described herein can be made primarily of polyimide. In other examples, it can be made of any of biocompatible polyimides, glass-reinforced epoxy laminate materials, copper, or graphene, alone or in combination. In some examples, the electrodes 160A described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc. The structure of the first flexible circuit 110 (and, likewise, the second flexible circuit 150) is discussed in further detail with respect to FIGs. 3A-6E below.

The end effector 100 can further include a framework 120 contiguous to the insulative material 130 or in the insulative material 130. In examples in which the distal tip 28 includes framework 120, the framework 120 can be disposed directly on the first flexible circuit 150 (or both the first flexible circuit 150 and the second flexible circuit 110) with none, or very little, of the insulative material 130 coming between the two. In other examples, insulative layers of the insulative material 130 can space the framework 120 from the flexible circuits 110, 150. In some examples, the framework 120 is disposed in the insulative material 130 and is substantially planar along the longitudinal axis 60 such that the longitudinal axis 60 is parallel to or coincident with the framework 120. In some examples, the framework 120 is symmetric relative to the longitudinal axis 60. In some examples, the framework 120 is formed from a flexible, resilient material. By way of example, the framework can be formed from a shape-memory alloy such as nickel-titanium, also known as Nitinol, cobalt chromium, stainless steel, and/or other alloys that exhibit pseudo-elastic and/or super-elastic properties. The framework 120 can be formed from a planar or cylindrical stock of material using any suitable method. For example, the framework 120 can be formed by cutting, laser cutting, stamping, etc.

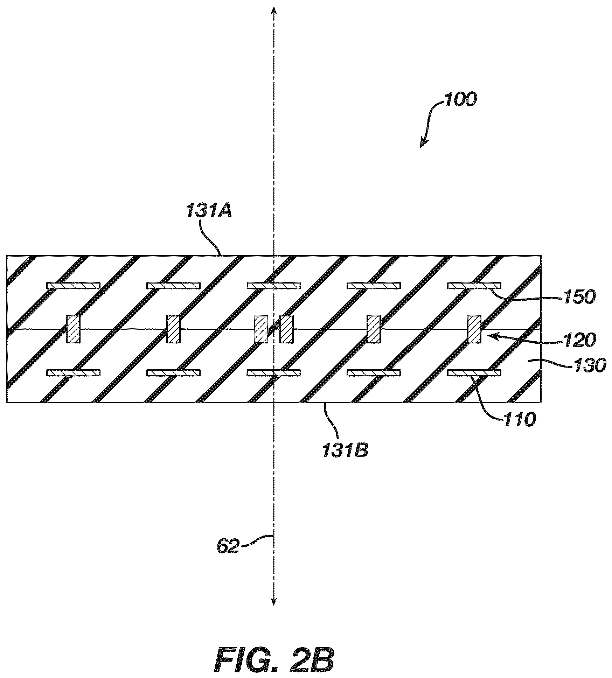

As mentioned above, the flexible circuits 110, 150 are disposed in or on an insulative material 130 that extends along the longitudinal axis 60. More specifically, the flexible circuits 110, 150 can have any number of positional relationships with the insulative material 130 without departing from the spirit and scope of the present disclosure. FIGs. 2B-2D, which are exemplary alternative cross-sectional views taken along line L1 in FIG. 2A when the end effector 100 is assembled, illustrate some of these potential positional relationships. These different positional relationships can provide various advantages depending on the selected design, such as improved mechanical, manufacturing, and/or sensing properties.

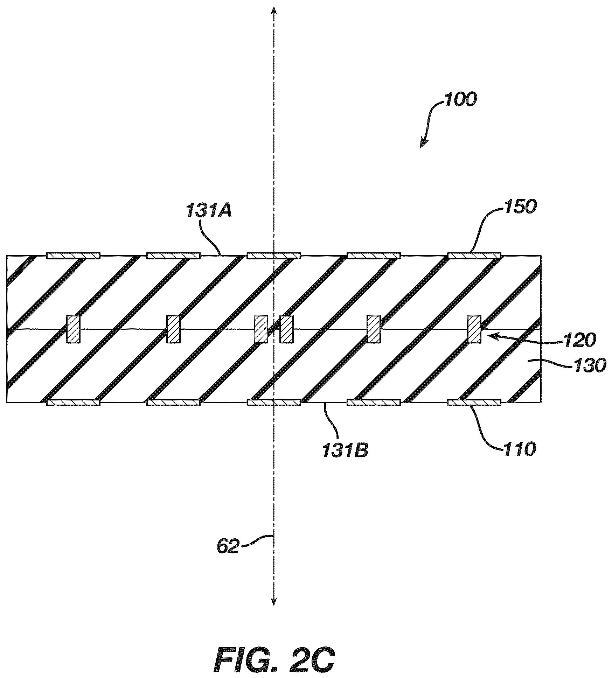

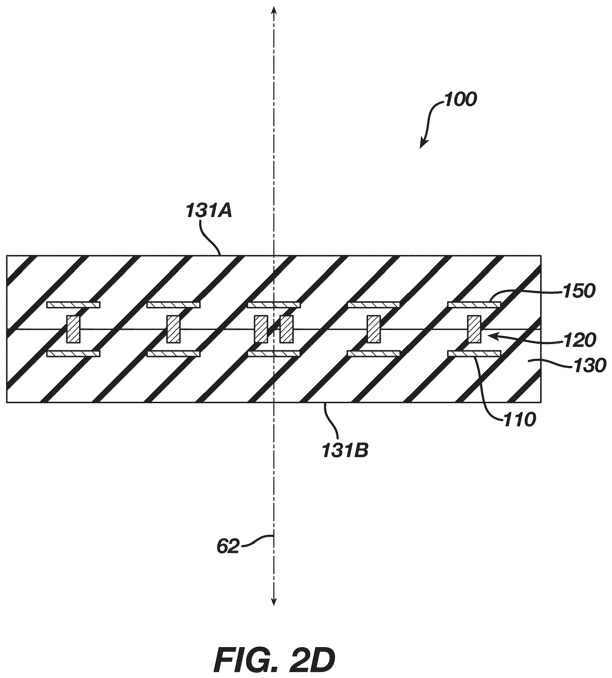

By way of non-limiting example, one or both of flexible circuits 110, 150 can be disposed (1) on outer surfaces 131A, 131B of the insulative material 130 (e.g., as seen in FIG. 2C), (2) suspended within the insulative material 130 and laying approximately flat (e.g., as seen FIG. 2B or FIG. 2D), or (3) suspended on and/or within the insulative material 130 at varying distances from the framework 120 (e. g., one or more portions of the flexible circuits 110, 150 can be disposed at one height (relative to vertical axis 62) to the framework 120 and other portion(s) of the flexible circuits 110, 150 can be disposed at another, different height (relative to the vertical axis) from the framework 120).

In other words, regarding the third example mentioned in the preceding paragraph, the flexible circuits 110, 150 do not necessarily need to lay flat and/or in a parallel plane as a plane of the framework 120 (i.e., it can be non-parallel with the framework 120). For example, some portions of the flexible circuits 110, 150 can be closer to the outer surfaces 131A, 131B of the insulative material 130 (e.g., a portion of the flexible circuits 110, 150 with a configuration like the depiction of FIG. 2B, which is further from the framework 120), while others can be closer to the framework 120 (e.g., a portion of the flexible circuits 110, 150 with a configuration like the depiction of FIG. 2D, which is closer to the framework).

The insulative material 130 can be contiguous to the contact surfaces of the electrodes 160 so that only the contact surfaces of at least a portion of the plurality of electrodes 160 are exposed to the ambient environment. As used herein, "ambient environment" refers to the external environment such as the organ in which the first end effector 100 is deployed or in the operating theater prior to being deployed in the biological organ. The insulative material 130 at least partially encapsulates and/or spaces the different layers of the end effector 100 (e.g., the flexible circuits 110, 150 and the framework 120) along the vertical axis 62.

It is noted that not all of the electrodes 160 on the flexible circuits 110, 150 described herein need be exposed through the insulative material 130 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood.

Insulative material 130 can include one or more sheets fused together proximate the framework 120 into a single, contiguous, generally planar insulative mass 130. This insulative material 130 also serves to enhance the atraumaticity of the end effector tip 100 and to protect the subject from sharp edges. The insulative material 130 can include polymer. The insulative material 130 can be heat formed around at least a portion of the first flexible circuit 150, the second flexible circuit 110, and the framework 120. The polymer can include TPU or other heat formed or shaped material which lends itself to said heat forming.

Furthermore, while the insulative material 130 is shown to be flat in these figures, insulative material 130 can be shaped, scalloped, ribbed, ridged, concaved, convexed, or otherwise configured such that the overall profile of insulative material 130 yields physical and/or mechanical properties, such as rigidity and flexion along multiple axes, required by the distal tip 28/end effector 100, mentioned above.

A flexible substrate of each flexible circuit 110, 150 (e.g., the flexible substrate 150-1 seen in FIG. 3B) comprises a bio-compatible material and has a first side and a second side. In some examples, the flexible substrate is formed entirely from or about entirely from the bio-compatible material. The electrodes 160 are disposed on a surface of the flexible substrate, such as a first surface 150A of the first flexible circuit 150. In some examples, the electrodes 160 are disposed on only one side of the substrate 150-1, with electrical interconnections/traces 162 that are connected and supply power thereto being routed on an opposite surface 150B of the flexible circuit 150. See, for example, FIG. 3B, as well as the phantom lines in FIG. 3A depicting exemplary electrical traces 162. Put another way, the electrodes 160 are directed so as to face away from the framework 120, with the electrical traces 162 being directed so as to face towards the framework 120.

With specific reference to FIGs. 4 and 5, each flexible circuit 110, 150 includes a plurality of electrodes 160, a base 111, 151 comprising a soldering pad region (bottom portion of FIGs. 5 and 4, respectively) disposed at a proximal end thereof, a plurality of tines 112-116, 152-156 extending from the base 111, 151, a plurality of voids 119A-H, 159A-H defined between the tines 112-116, 152-156, and a connecting distal segment 118, 158.

With specific reference to FIG. 4, the first flexible circuit 150 includes a flexible substrate 150-1 a central tine 154 that extends along the longitudinal axis 60 and offset tines 152, 153, 155, 156 that are offset from the longitudinal axis. More specifically, the offset tines 152, 153, 155, 156 extend from the base 151 away from the longitudinal axis 60 and then generally along/parallel thereto. Multiple electrodes 160 are disposed on each tine 152-156. The electrodes 160 can be disposed along the tines 152-156 such that they are aligned relative to electrodes 160 on adjacent tines 152-156 along the longitudinal axis 60. In other examples, the electrodes 160 can be unaligned (i.e., staggered) relative to electrodes 160 on adjacent tines in a direction transverse to the longitudinal axis 60 such that they are arranged in an alternatingly aligned pattern from tine to tine.

The tines 152-156 include a first tine 152 extending from the base 151 along the longitudinal axis 60, a second tine 153 extending from the base 151 along the longitudinal axis 60, a third tine 154 extending from the base 151 along the longitudinal axis 60, a fourth tine 155 extending from the base 151 along the longitudinal axis 60, and a fifth tine 156 extending from the base 151 along the longitudinal axis 60. As seen in FIG. 4, the first through fifth tines 152-156 are consecutively arranged from left to right. The first flexible circuit further includes a first intermediate connecting segment 157A connecting the second tine 153 and the third tine 154, a second intermediate connecting segment 157B connecting the third tine 154 and the fourth tine 155 towards a distal end of the flexible circuit 150, and a connecting distal segment 158 connecting distal ends of the first tine 152, the second tine 153, the third tine 154, the fourth tine 155, and the fifth tine 156.

The tines 152-156, connecting segments 157A, 157B and connecting distal segment 158 collective define respective voids 159A-H therebetween. Specifically, voids 159A-H (i.e., an area not covered by any material) are defined between the respective tines 152-156 of the flexible circuit 150. This reduction of material can aid in facilitating the collapsing of the end effector 100 into the sheath and/or insertion tool.

Additionally, the tines include connecting outer segments 152A, 156A that do not include electrodes 160. Rather, these segments aid in defining the shape of the end effector 100 and provide reinforcement/protection to the segments of the flexible circuit 150 that carries electrical traces 162 and/or electrodes 160.

Making continued reference to FIGs. 2-4 and 6A-6E, a plurality of sensing loops 140 can be provided that are disposed on the flexible substrate 150-1 of the first flexible circuit 150. Specifically, and as exemplified in FIG. 3B, the electrodes 160 and the location sensing loops 140 (see coils 142 of the location sensing loop 140) are disposed on the same first surface 150A of the flexible substrate 150-1.

Each sensing loop 140 functions as a position sensor (and is therefore also referred to herein as a location sensing loop, although it is noted that the loop can have more functions than providing positional information) that has one or more coils (each coil corresponding to a single turn of the respective location sensing loop) with traces that each, when subjected to a magnetic field, induce a current indicative of a position and/or shape of the end effector 100. Each coil comprises a surface area defined by the area enclosed by the respective coil and corresponds to a particular region of the flexible circuit 110, 150. By providing multiple coils in a sensing loop 140, the overall surface area covered by the loop 140 can be increased. Moreover, by providing the sensing loops 140 on the same layer of the end effector 100 as the electrodes 160, the electrode traces 162 can be shifted closer to the neutral plane of the end effector 100 (compared with an end effector where the sensing loops 140 require their own flexible circuit layer), which minimizes the strain on these traces 162 and reduces the complexity of the end effector 100.

In the example of the first flexible circuit 150, there are first and second location sensing loops 140A, 140B. The first location sensing loop 140A and the second location sensing loop 140B each includes one or more coils 142. The first location sensing loop 140A generally extends along the base 151, the first tine 152, the connecting distal segment 158, the second tine 153, the first intermediate connecting segment 157A, and the third tine 154. The second location sensing loop 140B generally extends along the base 151, the fifth tine 156, the connecting distal segment 158, the fourth tine 155, the second intermediate connecting segment 157B, and the third tine 154.

As seen in FIG. 4, the first location sensing loop 140A generally is divided into a first segment 140A-1 and a second segment 140A-2. The first segment 140A-1 is routed along the first tine 152 and a lateral portion of the distal connecting segment 158, while the second segment 140A-2 is routed along the third tine 154 and a first intermediate connecting segment 157A. These segments 140A-1, 140A-2 converge together on a distal portion 151A of the base 151 and on the second tine 153 (which is offset from the longitudinal axis 60).

Further to the above, in the present example, the first location sensing loop 140A includes three coils 142A, 142B, 142C (i.e., a first coil 142A, a second coil 142B, and a third coil 142C) that extend around the same region of the flexible circuit 150. However, other numbers of coils 142 may be employed without departing from the spirit and scope of the present disclosure (as discussed in greater detail below). FIGs. 6A-6E depict portions of the flexible circuit 150 demonstrating exemplary routing configurations of the coils 142A-142C. As seen in FIG. 6A, the first location sensing loop 140A extends from a first distal end 141A (which can comprise a soldering pad 141A) to a second distal end 141B (which can comprise a soldering pad 141B), both of which are proximal to one another on the base 151 of the flexible circuit 150.

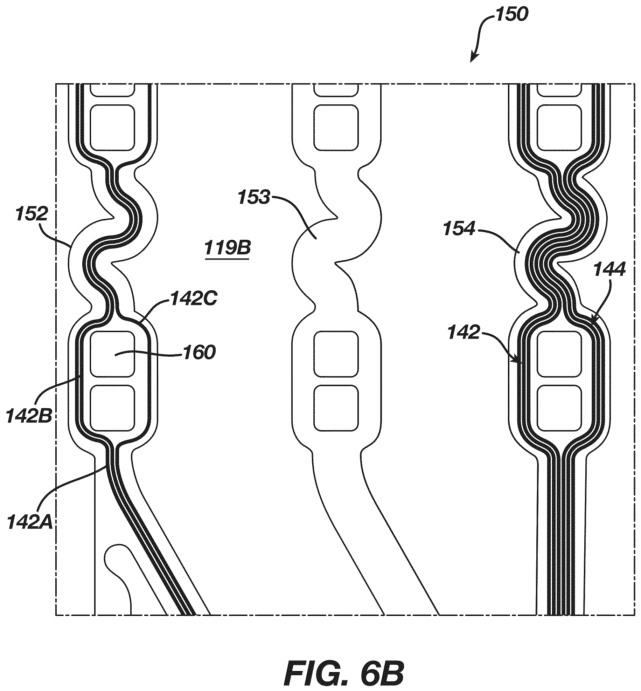

In order to prevent electrical shorting, the coils 142A-142C are routed so as to avoid contact with one another. This results in the location sensing loop 140A having a spiral shape, with the first soldering pad 141A being disposed on an exterior portion of the spiral shape on the first surface 150A of the flexible substrate 150-1, and the second soldering pad 141B being disposed on an interior portion of the spiral shape on the first surface 150A of the flexible substrate 150-1. See FIGs. 6A-6E for reference. As seen, e.g., in FIG. 6B, each of the coils 142A-142C are also routed such that they pass around and do not contact the electrodes 160 (to also prevent electrical shorting).

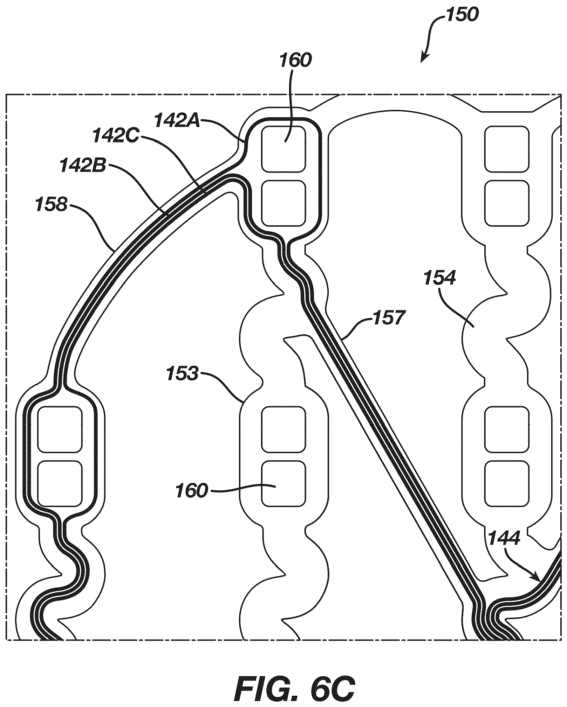

This routing can be employed in various configurations without departing from the spirit and scope of the present disclosure. For example, as seen in FIG. 6B, first coil 142A and second coil 142B pass by the electrodes 160 on the first tine 152 to the left, while the third coil 142 C passes by the electrodes 160 on the first tine 152 on the right. In contrast, on the third tine 154, the three coils 142A-142C pass by the electrodes 160 on the same side (which prevents crossing paths with the second location sensing loop 140B, which is discussed in greater detail below). As seen in FIG. 6C, around another electrode 160 (e.g., uppermost one on second tine 153), the first coil 142A can pass thereby on one side while the second and third coils 142B, 142C pass by the electrode 160 on the same side.

As seen in FIG. 4, the second location sensing loop 140B is symmetrical about the longitudinal axis 60 with the first location sensing loop 140 and generally is divided into a first segment 140B-1 and a second segment 140B-2. The first segment 140B-1 is routed along the fifth tine 156 and a lateral portion of the distal connecting segment 158, while the second segment 140B-2 is routed along the third tine 154 (generally parallel with the second segment 140A-2 of the first location sensing loop 140A) and the second intermediate connecting segment 157B. These segments 140B-1, 140B-2 converge together on the distal portion 151A of the base 151 and on the fourth tine 155 (which is offset from the longitudinal axis 60).

Further to the above, and similar to the first location sensing loop 140A, the second location sensing loop 140B includes three coils 144A, 144B, 144C (i.e., a first coil 144A, a second coil 144B, and a third coil 144C) that extend around the same region of the flexible circuit 150. However, other numbers of coils 144 may be employed without departing from the spirit and scope of the present disclosure (as discussed in greater detail below). As seen in FIG. 6A, the second location sensing loop 140A extends from a first distal end 141D (which can comprise a soldering pad 141D) to a second distal end 141C (which can comprise a soldering pad 141C), both of which are proximal to one another on the base 151 of the flexible circuit 150.

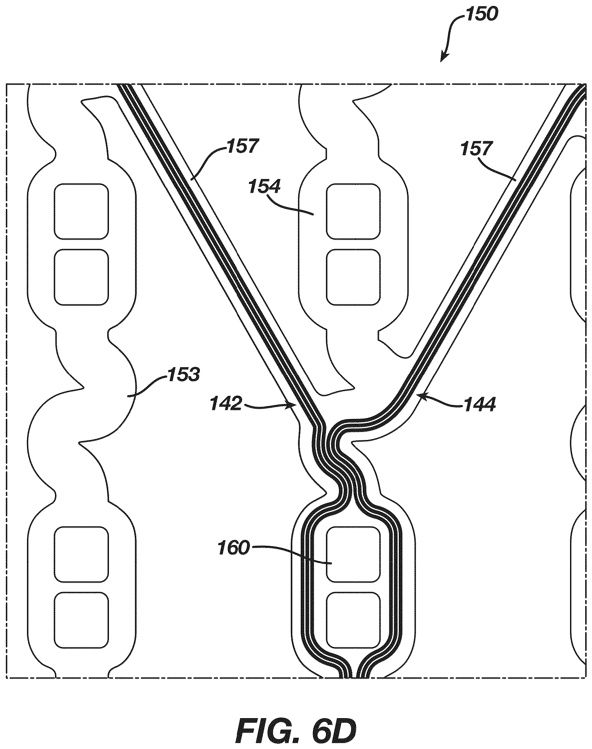

In order to prevent electrical shorting, like the first location sensing loop 140A, the coils 144A-144C are routed so as to avoid contact with one another. This results in the second location sensing loop 140B having a spiral shape, with the first soldering pad 141D being disposed on an exterior portion of the spiral shape on the first surface 150A of the flexible substrate 150-1, and the second soldering pad 141C being disposed on an interior portion of the spiral shape on the first surface 150A of the flexible substrate 150-1. See FIGs. 6A-6E for reference. As seen, e.g., in FIG. 6B, each of the coils 142A-142C are also routed such that they pass around and do not contact the electrodes 160 (to also prevent electrical shorting) along their respective paths. It is noted that the second location sensing loop 140B can take a generally mirrored path as that of the first location sensing loop 140A, or a different path, depending on the requirements of the design. As noted above, this routing can be employed in various configurations without departing from the spirit and scope of the present disclosure. As seen particularly in FIG. 6D, the first and second location sensing loops 140A, 140B converge along the connecting segments 157 together on the third tine 154.

Turning now to FIG. 5, the second flexible circuit 110 shares the same design concepts as that of the first flexible circuit 150, but with a different configuration of the location sensing loop 140. With specific reference to FIG. 5, the second flexible circuit 110 includes a flexible substrate 110-1 a central tine 114 that extends along the longitudinal axis 60 and offset tines 112, 113, 115, 116 that are offset from the longitudinal axis 60. More specifically, the offset tines 112, 113, 115, 116 extend from the base 111 away from the longitudinal axis 60 and then generally along/parallel thereto. Multiple electrodes 160 are disposed on each tine 112-116. The electrodes 160 can be disposed along the tines 112-116 such that they are aligned relative to electrodes 160 on adjacent tines 112-116 along the longitudinal axis 60. In other examples, the electrodes 160 can be unaligned (i.e., staggered) relative to electrodes 160 on adjacent tines in a direction transverse to the longitudinal axis 60 such that they are arranged in an alternatingly aligned pattern from tine to tine.

The tines 112-116 include a first tine 112 extending from the base 111 along the longitudinal axis 60, a second tine 113 extending from the base 111 along the longitudinal axis 60, a third tine 114 extending from the base along the longitudinal axis, a fourth tine 115 extending from the base along the longitudinal axis, and a fifth tine 116 extending from the base along the longitudinal axis. As seen in FIG. 5, the first through fifth tines 112-116 are consecutively arranged from left to right. The second flexible circuit 110 further includes a first intermediate connecting segment 117 connecting the second tine 113 and the third tine 114, a second intermediate connecting segment 117 connecting the third tine 114 and the fourth tine 115 towards a distal end of the flexible circuit 110, and a connecting distal segment 118 connecting distal ends of the first tine 112, the second tine 113, the third tine 114, the fourth tine 115, and the fifth tine 116.

The tines 112-116, connecting segments 117, and connecting distal segment 118 collective define respective voids 119A-H therebetween. This reduction of material can aid in facilitating the collapsing of the end effector 100 into the sheath and/or insertion tool.

Additionally, the tines 112-116 include connecting outer segments (lower left and right unlabeled regions in FIG. 5) that do not include electrodes 160. Rather, these segments aid in defining the shape of the end effector 100 and provide reinforcement/protection to the segments of the flexible circuit 110 that carries electrical traces 162 and/or electrodes 160.

It is noted that, like the previously described flexible circuit 150, not all of the electrodes 160 on the second flexible circuit 110 described herein need be exposed through the insulative material 130 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood.

Making continued reference to FIG. 5, a single location sensing loop 140C can be provided that are disposed on the flexible substrate 110-1 of the second flexible circuit 110. Specifically, and as exemplified by FIG. 3B 's depiction of the first flexible substrate 150-1, the electrodes 160 and the location sensing loop 140 (see coils 142 of the location sensing loop 140) are disposed on the same surface of the flexible substrate.

The location sensing loop 140C functions as a position sensor that has one or more coils (each coil corresponding to a single turn of the respective location sensing loop) with traces that each, when subjected to a magnetic field, induce a current indicative of a position and/or shape of the end effector 100. Each coil comprises a surface area defined by the area enclosed by the respective coil and corresponds to a particular region of the flexible circuit 150. By providing multiple coils in a location sensing loop 140, the overall surface area covered by the loop 140 can be increased. Moreover, by providing the location sensing loops 140 on the same layer of the end effector 100 as the electrodes 160, the electrode traces 162 can be shifted closer to the neutral plane of the end effector 100 (compared with an end effector where the sensing loops 140 require their own flexible circuit layer), which minimizes the strain on these traces 162 and reduces the complexity of the end effector 100.

In the example of the second flexible circuit 110, as mentioned above, there is a single location sensing loop 140C including one or more coils 146A-146C. The first location sensing loop 140A and the second location sensing loop 140B each includes one or more coils 142. The first location sensing loop 140A generally extends along the base 151, the first tine 152, the connecting distal segment 158, the second tine 153, the first intermediate connecting segment 157A, and the third tine 154. The second location sensing loop 140B generally extends along the base 151, the fifth tine 156, the connecting distal segment 158, the fourth tine 155, the second intermediate connecting segment 157B, and the third tine 154.

As seen in FIG. 4, the first location sensing loop 140A generally is divided, by the longitudinal axis 60, into a first segment 140C-1 and a second segment 140C-2. The first segment 140C-1 is routed along the second tine 112 (which is generally parallel to the longitudinal axis 60) and one of the connecting segments 117, while the second segment 140C-2 is routed along the fourth tine 115 (which is generally parallel to the longitudinal axis 60) and the other connecting segment 157. These segments 140C-1, 140C-2 converge together on a distal portion of the base 111 and on the third tine 114 (which, as mentioned above, is disposed on/along the longitudinal axis 60).

Further to the above, in the present example, the location sensing loop 140C includes three coils 146A, 146B, 146C (i.e., a first coil 146A, a second coil 146B, and a third coil 146C) that extend around the same region of the flexible circuit 110. However, other numbers of coils 146 may be employed without departing from the spirit and scope of the present disclosure (as discussed in greater detail below). While specific routing of the respective coils 146A-146C are not explicitly depicted, those skilled in the art will appreciate that the location sensing loop 140C of the second flexible circuit 110 can employ the same routing principles as those previously described, as well as the same design concepts, such as that as the spiraling shape (that connects at its distal ends to solder pads 141E, 141F).

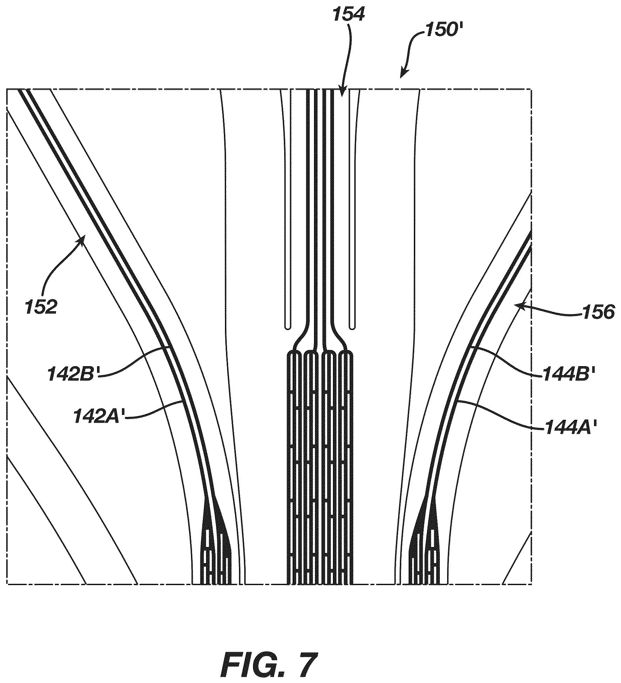

Turning briefly to FIG. 7, and as discussed above, the presently disclosed technology can employ different numbers of coils without departing from the spirit and scope of the present disclosure. For example, and alternative flexible circuit 150' can include first and second location sensing loops that each include two coils 142A', 142B' and 144A', 144B' respectively, rather than three coils.

The present disclosure provides a medical probe assembly 200 as shown in FIG. 8 which can include a tubular member 230 extending along a longitudinal axis 60 and configured to deliver end effector 100 to and out of a sheath 210. A physician 24 can manipulate the catheter assembly 200 with handle 220. Appropriate examples for catheter assembly 200 and its subcomponents such as handle 220, sheath 210, tubular member 230, and others not mentioned herein are described in US Patent publication No. 2021/0369339, which is incorporated herein by reference.

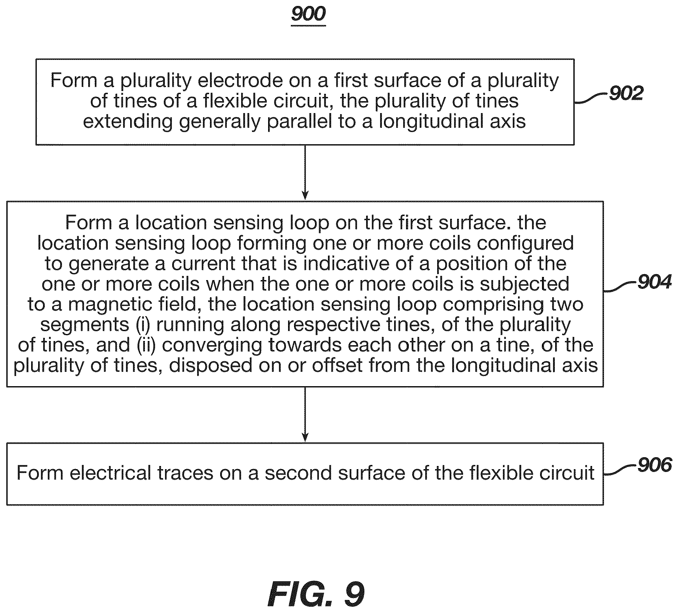

Further to the above-described examples, and with reference to FIG. 9, a method 900 of manufacturing an end effector for a medical device can include the following. The method includes forming 902 a plurality of electrodes on a first surface of a plurality of tines of a flexible circuit, the plurality of tines extending generally parallel to a longitudinal axis. The first surface is common across the tines and is of the flexible substrate discussed above. The method includes forming 904 a location sensing loop on the first surface. The location sensing loop form one or more coils configured to generate a current that is indicative of a position of the one or more coils when the one or more coils is subjected to a magnetic field. The location sensing loop comprises two segments (i) running along respective tines, of the plurality of tines, and (ii) converging towards each other on a tine, of the plurality of tines, disposed on or offset from the longitudinal axis. The location sensing loop is routed such that it does not contact the electrode. The location sensing loop is routed in a spiral shape. The method further includes forming 906 electrical traces on a second surface of the flexible circuit. As discussed above, by forming the position sensing loop on the same flexible circuit as that of the electrodes, the total number of layers of flexible circuit in the end effector 100 can be reduced,

The disclosed technology described herein can be further understood according to the following clauses:
Clause 1. A flexible circuit for an end effector of a medical probe, the flexible circuit comprising: a flexible substrate defining a plurality of tines extending along a longitudinal axis; a plurality of electrodes disposed on each of the plurality of tines; and a first sensing loop disposed on the flexible substrate, the first location sensing loop forming one or more coils configured to generate a current that is indicative of a position of the one or more coils when the one or more coils is subjected to a magnetic field, and the first location sensing loop comprising two segments disposed on respective tines, of the plurality of tines, extending generally parallel to the longitudinal axis and converging towards each other on a tine, of the plurality of tines, disposed on the longitudinal axis.
Clause 2. The flexible circuit of clause 1, the flexible substrate comprising a first surface and a second surface, the electrode and the one or more sensing loops both being disposed on the first surface.
Clause 3. The flexible circuit of clause 2, further comprising one or more electrical interconnections electrically connected to the electrode and disposed along the second surface.
Clause 4. The flexible circuit of any one of clauses 2-3, the one or more coils being routed to pass around each electrode of the plurality of electrodes along a path of the one of more coils and along the flexible substrate.
Clause 5. The flexible circuit of any one of clauses 1-4, the at least one coil comprising a first coil extending around a region of the flexible substrate and a second coil extending around the region of the flexible substrate.
Clause 6. The flexible circuit of clause 5, the at least one coil comprising a third coil extending around the region of the flexible substrate.
Clause 7. The flexible circuit of clause 6, the first coil, the second coil, and the third coil being routed along the flexible substrate such that the first coil and the second coil pass around the electrode on a first side of the electrode and the third coil passes around the electrode on an opposing second side of the electrode.
Clause 8. The flexible circuit of clause 6, the first coil, the second coil, and the third coil being routed along the flexible substrate such that the first coil, the second coil, and the third coil pass around the electrode on a same side of the electrode.
Clause 9. The flexible circuit of any one of clauses 1-8, each location sensing loop extending from a first distal end to a second distal end.
Clause 10. The flexible circuit of clause 9, the first distal end and the second distal end being disposed at a proximal end of the flexible circuit.
Clause 11. The flexible circuit of any one of clauses 9-10, the first distal end comprising a first soldering pad and the second distal end comprising a second soldering pad.
Clause 12. The flexible circuit of clause 11, the flexible substrate comprising a base, the first soldering pad and the second soldering pad being disposed on the base.
Clause 13. The flexible circuit of any one of clauses 11-12, each location sensing loop comprising a spiral shape that defines the one or more coils, with the first soldering pad being disposed on an exterior portion of the spiral shape on a first surface of the flexible substrate, and the second soldering pad is disposed on an interior portion of the spiral shape on the first surface of the flexible substrate.
Clause 14. The flexible circuit of any one of clauses 9-12, each location sensing loop comprising a spiral shape that defines the one or more coils.
Clause 15. The flexible circuit of clause 14, each coil corresponding to a single turn of the respective location sensing loop.
Clause 16. The flexible circuit of any one of clauses 1-15, the flexible substrate comprising a base, a first intermediate connecting segment, a second intermediate connecting segment, and a connecting distal segment, and the plurality of tines comprising: a first tine extending from the base along the longitudinal axis; a second tine extending from the base along the longitudinal axis; a third tine extending from the base along the longitudinal axis, the tine where the two segments converge towards each other corresponding to the third tine; a fourth tine extending from the base along the longitudinal axis; and a fifth tine extending from the base along the longitudinal axis, the first intermediate connecting segment connecting the second tine and the third tine; the second intermediate connecting segment connecting the third tine and the fourth tine, and the connecting distal segment connecting distal ends of the first tine, the second tine, the third tine, the fourth tine, and the fifth tine.
Clause 17. The flexible circuit of clause 16, the first tine, the second tine, the third tine, the fourth tine, the fifth tine, the first intermediate connecting segment, and the second intermediate connecting segment defining respective voids therebetween.
Clause 18. The flexible circuit of any one of clauses 16-17, the first location sensing loop extending along the base, the second tine, the first intermediate connecting segment, the third tine, the second intermediate connecting segment, and the fourth tine, with the two segments converging towards each other on the third tine and on the base.
Clause 19. The flexible circuit of any one of clauses 1-18, each coil comprising a surface area defined by an area enclosed the respective coil, each area corresponding to a region of the flexible circuit.
Clause 20. The flexible circuit of any one of clauses 1-19, the current being indicative of a shape of a portion of the flexible circuit.
Clause 21. A flexible circuit for an end effector of a medical probe, the flexible circuit comprising: a flexible substrate defining a plurality of tines extending along a longitudinal axis; a plurality of electrodes disposed on each of the plurality of tines; and first and second sensing loops disposed on the flexible substrate, each of the first and second location sensing loop forming one or more coils configured to generate a current that is indicative of a position of the one or more coils when the one or more coils is subjected to a magnetic field, the first location sensing loop comprising two segments disposed on respective tines, of the plurality of tines, extending along the longitudinal axis and converging towards each other on a tine, of the plurality of tines, offset from the longitudinal axis, and the second location sensing loop comprising two segments disposed on respective tines, of the plurality of tines, extending along the longitudinal axis and converging towards each other on another tine, of the plurality of tines, offset from the longitudinal axis.
Clause 22. The flexible circuit of clause 21, the flexible substrate comprising a first surface and a second surface, the electrode and the one or more sensing loops both being disposed on the first surface.
Clause 23. The flexible circuit of clause 22, further comprising one or more electrical interconnections electrically connected to the electrode and disposed along the second surface.
Clause 24. The flexible circuit of any one of clauses 22-23, the one or more coils being routed to pass around the electrode along the flexible substrate.
Clause 25. The flexible circuit of any one of clauses 21-24, the at least one coil comprising a first coil extending around a region of the flexible substrate and a second coil extending around the region of the flexible substrate.
Clause 26. The flexible circuit of clause 25, the at least one coil comprising a third coil extending around the region of the flexible substrate.
Clause 27. The flexible circuit of clause 26, the first coil, the second coil, and the third coil being routed along the flexible substrate such that the first coil and the second coil pass around a first electrode, of the plurality of electrodes, on a first side of the first electrode and the third coil passes around the first electrode on an opposing second side of the first electrode.
Clause 28. The flexible circuit of clause 26, the first coil, the second coil, and the third coil being routed along the flexible substrate such that the first coil, the second coil, and the third coil pass around a first electrode, of the plurality of electrodes, on a same side of the first electrode.
Clause 29. The flexible circuit of any one of clauses 21-28, each location sensing loop extending from a first distal end to a second distal end.
Clause 30. The flexible circuit of clause 29, the first distal end and the second distal end being disposed at a proximal end of the flexible circuit.
Clause 31. The flexible circuit of any one of clauses 29-30, the first distal end comprising a first soldering pad and the second distal end comprising a second soldering pad.
Clause 32. The flexible circuit of clause 31, the flexible substrate comprising a base, the first soldering pad and the second soldering pad being disposed on the base.
Clause 33. The flexible circuit of any one of clauses 31-32, each location sensing loop comprising a spiral shape that defines the one or more coils, with the first soldering pad being disposed on an exterior portion of the spiral shape on a first surface of the flexible substrate, and the second soldering pad is disposed on an interior portion of the spiral shape on the first surface of the flexible substrate.
Clause 34. The flexible circuit of any one of clauses 29-32, each location sensing loop comprising a spiral shape that defines the one or more coils.
Clause 35. The flexible circuit of clause 34, each coil corresponding to a single turn of the respective location sensing loop.
Clause 36. The flexible circuit of any one of clauses 21-35, the flexible substrate comprising a base, a first intermediate connecting segment, a second intermediate connecting segment, and a connecting distal segment, and the plurality of tines comprising: a first tine extending from the base along the longitudinal axis; a second tine extending from the base along the longitudinal axis, the tine where the two segments of the first location sensing loop converge towards each other corresponding to the second tine; a third tine extending from the base along the longitudinal axis; a fourth tine extending from the base along the longitudinal axis, the another tine where the two segments of the second sensing loop converge towards each other corresponding to the fourth tine; and a fifth tine extending from the base along the longitudinal axis, the first intermediate connecting segment connecting the second tine and the third tine; the second intermediate connecting segment connecting the third tine and the fourth tine, and the connecting distal segment connecting distal ends of the first tine, the second tine, the third tine, the fourth tine, and the fifth tine.
Clause 37. The flexible circuit of clause 36, the first tine, the second tine, the third tine, the fourth tine, the fifth tine, the first intermediate connecting segment, and the second intermediate connecting segment defining respective voids therebetween.
Clause 38. The flexible circuit of any one of clauses 36-37, the first location sensing loop extending along the base, the first tine, the connecting distal segment, the second tine, the first intermediate connecting segment, and the third tine.
Clause 39. The flexible circuit of any one of clauses 36-38, the second sensing loop extending along the base, the fifth tine, the connecting distal segment, the fourth tine, the second intermediate connecting segment, and the third tine.
Clause 40. The flexible circuit of any one of clauses 21-39, each coil comprising a surface area defined by an area enclosed the respective coil, each area corresponding to a region of the flexible circuit.
Clause 41. The flexible circuit of any one of clauses 21-40, the current being indicative of a shape of a portion of the flexible circuit.
Clause 42. An end effector for a medical probe, the end effector comprising: an insulative material; a framework disposed in the insulative material, the framework being approximately planar along a longitudinal axis; and a first flexible circuit disposed in the insulative material such that the first flexible circuit is spaced apart from the framework along a vertical axis, the first flexible circuit comprising: a first flexible substrate defining a first plurality of tines extending along the longitudinal axis; a first plurality of electrodes disposed on each of the first plurality of tines; and first and second sensing loops disposed on the first flexible substrate, each of the first and second sensing loop forming one or more coils configured to generate a current that is indicative of a position of the one or more coils when the one or more coils is subjected to a magnetic field, the first location sensing loop comprising two segments disposed on respective tines, of the first plurality of tines, extending along the longitudinal axis and converging towards each other on a tine, of the first plurality of tines, offset from the longitudinal axis, and the second sensing loop comprising two segments disposed on respective tines, of the first plurality of tines, extending along the longitudinal axis and converging towards each other on another tine, of the first plurality of tines, offset from the longitudinal axis.
Clause 43. The end effector of clause 42, the first sensing loop extending on a first side of the first flexible circuit, and the second sensing loop extending on a second side of the first flexible circuit, the second side location sensing loop being disposed generally symmetrical about the longitudinal axis relative to the first side location sensing loop.
Clause 44. The end effector of any one of clauses 42-43, further comprising: a second flexible circuit disposed in the insulative material such that the second flexible circuit is spaced apart from the framework and the first flexible circuit along the vertical axis, the second flexible circuit comprising: a second flexible substrate defining a second plurality of tines extending along the longitudinal axis; a second plurality of electrodes disposed on each of the second plurality of tines; and a third sensing loop disposed on the second flexible substrate, the third location sensing loop forming one or more coils configured to generate a current that is indicative of a position of the one or more coils when the one or more coils is subjected to a magnetic field, and the third location sensing loop comprising two segments disposed on respective tines, of the second plurality of tines, extending generally parallel to the longitudinal axis and converging towards each other on a first tine, of the second plurality of tines, disposed on the longitudinal axis.
Clause 45. The end effector of clause 44, the third location sensing loop being disposed generally symmetrical about the longitudinal axis.
Clause 46. A method comprising: forming a plurality of electrodes on a first surface of a plurality of tines of a flexible circuit, the plurality of tines extending generally parallel to a longitudinal axis; and forming a location sensing loop on the first surface, the location sensing loop forming one or more coils configured to generate a current that is indicative of a position of the one or more coils when the one or more coils is subjected to a magnetic field, the location sensing loop comprising two segments (i) running along respective tines, of the plurality of tines, and (ii) converging towards each other on a tine, of the plurality of tines, disposed on or offset from the longitudinal axis.
Clause 47. The method of clause 46, the forming the location sensing loop comprising routing the location sensing loop such that the location sensing loop does not contact the electrode.
Clause 48. The method of any one of clauses 46-47, the forming the location sensing loop comprising routing the location sensing loop in a spiral shape.
Clause 49. The method of any one of clauses 46-48, further comprising: forming electrical traces on a second surface of the flexible circuit.
Clause 50. An end effector for a medical probe, the end effector comprising: an insulative material; a framework disposed in the insulative material, the framework being approximately planar along a longitudinal axis; and a first flexible circuit disposed in or on the insulative material, the first flexible circuit comprising: a first flexible substrate defining a first plurality of tines extending along the longitudinal axis; a first plurality of electrodes disposed on each of the first plurality of tines; and first and second sensing loops disposed on the first flexible substrate, each of the first and second sensing loop forming one or more coils configured to generate a current that is indicative of a position of the one or more coils when the one or more coils is subjected to a magnetic field, the first location sensing loop comprising two segments disposed on respective tines, of the first plurality of tines, extending along the longitudinal axis and converging towards each other on a tine, of the first plurality of tines, offset from the longitudinal axis, and the second sensing loop comprising two segments disposed on respective tines, of the first plurality of tines, extending along the longitudinal axis and converging towards each other on another tine, of the first plurality of tines, offset from the longitudinal axis.
Clause 51. The end effector of clause 50, the first flexible circuit being approximately planar along the longitudinal axis.
Clause 52. The end effector of clause 51, the first flexible circuit extending approximately parallel to the framework.
Clause 53. The end effector of any one of clauses 50-51, the first flexible circuit comprising a first portion and a second portion along the longitudinal axis, the first portion being disposed a first distance from the framework along a vertical axis, and the second portion being disposed a second distance from the framework along the vertical axis, the second distance being different from the first distance.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A flexible circuit for an end effector of a medical probe, the flexible circuit comprising:
a flexible substrate defining a plurality of tines extending along a longitudinal axis;
a plurality of electrodes disposed on each of the plurality of tines; and
a first sensing loop disposed on the flexible substrate,
the first sensing loop forming one or more coils configured to generate a current that is indicative of a position of the one or more coils when the one or more coils is subjected to a magnetic field, and
the first sensing loop comprising two segments disposed on respective tines, of the plurality of tines, extending generally parallel to the longitudinal axis and converging towards each other on a tine, of the plurality of tines, disposed on the longitudinal axis.

2. The flexible circuit of claim 1, the flexible substrate comprising a first surface and a second surface, the plurality of electrodes and the one or more sensing loops both being disposed on the first surface.

3. The flexible circuit of claim 2, further comprising one or more electrical interconnections electrically connected to the plurality of electrodes and disposed along the second surface.

4. The flexible circuit of claim 2 or 3, the one or more coils being routed to pass around each electrode of the plurality of electrodes along a path of the one of more coils and along the flexible substrate.

5. The flexible circuit of claim 1, the one or more coils comprising a first coil extending around a region of the flexible substrate and a second coil extending around the region of the flexible substrate, optionally the one or more coils comprising a third coil extending around the region of the flexible substrate, further optionally the first coil, the second coil, and the third coil being routed along the flexible substrate such that the first coil, the second coil, and the third coil pass around a first electrode, of the plurality of electrodes, on a same side of the first electrode.

6. The flexible circuit of any preceding claim, each location sensing loop extending from a first distal end to a second distal end.

7. The flexible circuit of claim 6, the first distal end and the second distal end being disposed at a proximal end of the flexible circuit, and/or the first distal end comprising a first soldering pad and the second distal end comprising a second soldering pad, optionally each location sensing loop comprising a spiral shape that defines the one or more coils, with the first soldering pad being disposed on an exterior portion of the spiral shape on a first surface of the flexible substrate, and the second soldering pad is disposed on an interior portion of the spiral shape on the first surface of the flexible substrate.

8. The flexible circuit of claim 6, each location sensing loop comprising a spiral shape that defines the one or more coils.

9. The flexible circuit of preceding claim, the flexible substrate comprising a base, a first intermediate connecting segment, a second intermediate connecting segment, and a connecting distal segment, and the plurality of tines comprising:
a first tine extending from the base along the longitudinal axis;
a second tine extending from the base along the longitudinal axis;
a third tine extending from the base along the longitudinal axis, the tine where the two segments converge towards each other corresponding to the third tine;
a fourth tine extending from the base along the longitudinal axis; and
a fifth tine extending from the base along the longitudinal axis,
the first intermediate connecting segment connecting the second tine and the third tine;
the second intermediate connecting segment connecting the third tine and the fourth tine, and
the connecting distal segment connecting distal ends of the first tine, the second tine, the third tine, the fourth tine, and the fifth tine.

10. The flexible circuit of claim 9, the first sensing loop extending along the base, the second tine, the first intermediate connecting segment, the third tine, the second intermediate connecting segment, and the fourth tine, with the two segments converging towards each other on the third tine and on the base.

11. The flexible circuit of any preceding claim, each coil comprising a surface area defined by an area enclosed the respective coil, each area corresponding to a region of the flexible circuit.

12. A flexible circuit for an end effector of a medical probe, the flexible circuit comprising:
a flexible substrate defining a plurality of tines extending along a longitudinal axis;
a plurality of electrodes disposed on each of the plurality of tines; and
first and second sensing loops disposed on the flexible substrate,
each of the first and second sensing loop forming one or more coils configured to generate a current that is indicative of a position of the one or more coils when the one or more coils is subjected to a magnetic field,
the first sensing loop comprising two segments disposed on respective tines, of the plurality of tines, extending along the longitudinal axis and converging towards each other on a tine, of the plurality of tines, offset from the longitudinal axis, and
the second sensing loop comprising two segments disposed on respective tines, of the plurality of tines, extending along the longitudinal axis and converging towards each other on another tine, of the plurality of tines, offset from the longitudinal axis.

13. The flexible circuit of claim 12, the flexible substrate comprising a base, a first intermediate connecting segment, a second intermediate connecting segment, and a connecting distal segment, and the plurality of tines comprising:
a first tine extending from the base along the longitudinal axis;
a second tine extending from the base along the longitudinal axis, the tine where the two segments of the first sensing loop converge towards each other corresponding to the second tine;
a third tine extending from the base along the longitudinal axis;
a fourth tine extending from the base along the longitudinal axis, the another tine where the two segments of the second sensing loop converge towards each other corresponding to the fourth tine; and
a fifth tine extending from the base along the longitudinal axis,
the first intermediate connecting segment connecting the second tine and the third tine;
the second intermediate connecting segment connecting the third tine and the fourth tine, and
the connecting distal segment connecting distal ends of the first tine, the second tine, the third tine, the fourth tine, and the fifth tine.

14. The flexible circuit of claim 13, (i) the first sensing loop extending along the base, the first tine, the connecting distal segment, the second tine, the first intermediate connecting segment, and the third tine, or (ii) the second sensing loop extending along the base, the fifth tine, the connecting distal segment, the fourth tine, the second intermediate connecting segment, and the third tine.

15. An end effector for a medical probe, the end effector comprising:
an insulative material;
a framework disposed in the insulative material, the framework being approximately planar along a longitudinal axis; and
a first flexible circuit disposed in the insulative material such that the first flexible circuit is spaced apart from the framework along a vertical axis, the first flexible circuit comprising:
a first flexible substrate defining a first plurality of tines extending along the longitudinal axis;
a first plurality of electrodes disposed on each of the first plurality of tines; and
first and second sensing loops disposed on the first flexible substrate,
each of the first and second sensing loop forming one or more coils configured to generate a current that is indicative of a position of the one or more coils when the one or more coils is subjected to a magnetic field,
the first sensing loop comprising two segments disposed on respective tines, of the first plurality of tines, extending along the longitudinal axis and converging towards each other on a tine, of the first plurality of tines, offset from the longitudinal axis, and
the second sensing loop comprising two segments disposed on respective tines, of the first plurality of tines, extending along the longitudinal axis and converging towards each other on another tine, of the first plurality of tines, offset from the longitudinal axis.
